# EUROPEAN PATENT SPECIFICATION

(11) **EP 3 493 859 B1**
(45) Date of publication and mention of the grant of the patent: **07.07.2021**
(21) Application number: 17757816.8
(22) Date of filing: 01.08.2017
(51) Int. Cl.: A61L 31/04, A61L 31/14

(54) **PHOTOINITIATING POLYMERISABLE COMPOSITION**
PHOTOINITIIIERENDE POLYMERISIERBARE ZUSAMMENSETZUNG
COMPOSITION POLYMÉRISABLE PHOTOAMORÇANTE

(30) Priority: 05.08.2016 GB 201613540
(43) Date of publication of application: 12.06.2019
(73) Proprietor: Lancaster University Business Enterprises Limited, Lancaster LA1 4YW (GB)
(72) Inventor: HARDY, John George, Lancaster Lancashire LA1 4YB (GB)
(74) Representative: Harrison IP Limited
(86) International application number: PCT/GB2017/052235
(87) International publication number: WO 2018/025026

(56) References cited:
- US-A1- 2006 276 860
- US-A1- 2007 032 846
- ANTON MIHIC ET AL: "A Conductive Polymer Hydrogel Supports Cell Electrical Signaling and Improves Cardiac Function After Implantation into Myocardial Infarct", CIRCULATION, vol. 132, no. 8, 24 August 2015 (2015-08-24), pages 772-784, XP55421370, US ISSN: 0009-7322, DOI: 10.1161/CIRCULATIONAHA.114.014937

## Description

The present invention relates to an injectable composition. There is also provided a method of producing a visual marking on the exterior of a human or animal body including the implantation of the composition to a human or animal body, and the subsequent polymerization of the composition within the human or animal body.

### BACKGROUND TO THE INVENTION

It is known to use multi-photon photo-polymerization *ex vivo* to build a structure modelled on a biological organ (for example see US2013197668A1). A solid block of gel containing monomers and a 2-photon active photo-initiator is prepared as a raw material. The shape of a desired object is traced out by the control of laser beams, optionally there may be additional steps such as a fixing step, and then excess gel is washed away to leave the desired structure.

In one type of photo-polymerization, light is absorbed directly by a reactant monomer. In a second type of photo-polymerization, light is absorbed by a photo-initiator material which absorbs the light and then transfers energy to the monomer to effect polymerization.

It is known to use multi-photon techniques destructively to induce controlled release of drugs from a structure *in vivo* (see for example US2003191458A1).

US2007/032846 describes methods and systems for treating skin for aesthetic, functional, health or other purposes are described. According to various embodiments, materials are delivered to or formed in or on the skin at multiple depths or heights in a pattern to form a hologram in or on the skin.

Anton Mihic et al. "A Conductive Polymer Hydrogel Supports Cell Electrical Signalling and Improves Cardiac Function after Implantation into Myocardial Infarct" (Circulation, vol. 132, no. 8, 24 August 2015, pages 772-784 XP55421370) discloses the injection of a conductive polymer hydrogel, and its use in maintaining heart function after myocardial infarction.

US2006/276860 discloses the use of photoresponsive materials and light, delivered in a controlled fashion to produce a patterned distribution of a material in the skin.

Currently the provision of structures *in vivo* requires incision and implantation. The present invention is advantageous because it permits *in situ in vivo* fabrication of structures and may require as few as one implantation (suitably one injection) of the precursor materials.

### SUMMARY OF INVENTION

The scope of protection is defined by the claims.

A method of producing a visual marking on the exterior of a human or animal body comprising:
implanting a composition at a site in the human or animal body 1 cm or less from the exterior of the human or animal body wherein the composition comprises a polymerisable precursor and a photo-initiator wherein the photo-initiator causes the polymerisable precursor to polymerise where the photo-initiator is in its excited state;
illuminating the site with electromagnetic radiation having a first wavelength of 400 to 1600 nm, wherein absorption of two or more photons of the electromagnetic radiation excites the photo-initiator causing initiation of polymerisation of the polymerisable precursor to form a polymer within the human or animal body, wherein the polymer is visible on the exterior of the human or animal body;
wherein the resultant polymer comprises, consists or consists essentially of one or more of the group consisting of poly(fluorene)s, polyphenylenes, polypyrenes, polyazulenes, polynaphthalenes, Poly(acetylene)s, Poly(p-phenylene vinylene), poly(pyrrole)s, polycarbazoles, polyindoles, polyazepines, poly(thiophene)s, poly(3,4-ethylenedioxythiophene), poly(p-phenylene sulfide), polyanilines and polyfurans; and
wherein the resultant polymer has an electrical conductivity of 10⁻¹⁰ S/cm or more.

According to one embodiment the resultant polymer has an electrical conductivity of 10⁻¹⁰ S/cm to 10⁴ S/cm.

Generally, polymerisation causes a change in physical state of the polymerizable precursor. Suitably, the change in physical state involves solidification of the polymerisable precursor. Alternatively, the viscosity of the polymerisable precursor may be greatly increased, for example to yield a gel (including a hydrogel), paste, foam or plastic.

Generally, the visual marking is in the form of a tattoo.

According to an aspect of the present invention, there is provided an injectable composition comprising a polymerisable precursor and a photo-initiator wherein the photo-initiator causes the polymerisable precursor to polymerise where the photo-initiator is in its excited state,
wherein absorption of two or more photons of electromagnetic radiation having a wavelength of 400 to 1600 nm excites the photo-initiator causing initiation of polymerisation of the polymerisable precursor,
wherein the resultant polymer comprises, consists or consists essentially of one or more of the group consisting of poly(fluorene)s, polyphenylenes, polypyrenes, polyazulenes, polynaphthalenes, poly(acetylene)s, poly(p-phenylene vinylene), poly(pyrrole)s, polycarbazoles, polyindoles, polyazepines, poly(thiophene)s, poly(3,4-ethylenedioxythiophene), poly(p-phenylene sulfide), polyanilines and polyfurans,
wherein the resultant polymer has an associated electrical conductivity of at least 10⁻¹⁰ S/cm.

According to one embodiment the resultant polymer has an electrical conductivity of 10⁻¹⁰ S/cm to 10⁴ S/cm.

The injectable composition may comprise a buffered aqueous based composition comprising monomers that are derivatives of pyrrole, aniline, thiophene, 3,4-ethylenedioxythiophene, fluorene, phenylene, pyrene, azulene, napthalene, indole, azepine, p-phenylene sulfide, p-phenylene vinylene, and/or furans; and moreover, a photo-initiator, and dye(s).

According to a further aspect of the present invention the injectable composition may comprise a buffered aqueous based composition comprising pyrrole, a photo-initiator (generally irgacure) and a dye.

Also disclosed is a kit, where the kit includes one or more of the compositions of the present teachings, or the components necessary to form the compositions of the present invention, and instructions for use thereof. Generally, the kit would comprise an energy source.

Also disclosed is a system for performing the methods disclosed herein. The system can include the composition as disclosed herein and one or more energy sources, typically lasers. The system may include an analytical instrument used to measure the wavelength and intensity of the energy source(s), and/or to measure the amount of composition implanted in the human or animal body. The system also can include a suitably programmed computer for carrying out one or more steps of the methods. For example, the suitably programmed computer can carry out or assist in one or more of control of the intensity of the energy source(s), measuring any fluctuations in the intensity of the energy source(s), and equivalents thereof.

Throughout the Application, where compositions are described as having, including, or comprising specific components, or where processes are described as having, including, or comprising specific process steps, it is contemplated that compositions of the present teachings also consist essentially of, or consist of, the recited components, and that the processes of the present teachings also consist essentially of, or consist of, the recited process steps.

In the Application, where an element or component is said to be included in and/or selected from a list of recited elements or components, it should be understood that the element or component can be any one of the recited elements or components, or the element or component can be selected from a group consisting of two or more of the recited elements or components.

The use of the terms "include," "includes", "including," "have," "has," or "having" should be generally understood as open-ended and non-limiting unless specifically stated otherwise.

The use of the singular herein includes the plural (and vice versa) unless specifically stated otherwise. In addition, where the use of the term "about" is before a quantitative value, the present teachings also include the specific quantitative value itself, unless specifically stated otherwise. As used herein, the term "about" refers to a ±10% variation from the nominal value unless otherwise indicated or inferred.

It should be understood that the order of steps or order for performing certain actions is immaterial so long as the present teachings remain operable. Moreover, two or more steps or actions may be conducted simultaneously.

As used herein, "pharmaceutically acceptable" refers to a substance that is acceptable for use in pharmaceutical applications from a toxicological perspective and does not adversely interact with the active ingredient. Accordingly, pharmaceutically acceptable carriers are those that are compatible with the other ingredients in the formulation and are biologically acceptable. Supplementary active ingredients can also be incorporated into the pharmaceutical compositions.

The methods and compositions described herein may be used in connection with a human, animal, fish or bird, in particular a mammal, such as a human although mention may also be made of primates, dogs, cats, rats, horses, camels, farm livestock such as cattle sheep, pigs, goats, deer; birds including chicken, geese, birds of prey and endangered animals including endangered birds.

As used herein, a "compound" refers to the compound itself and its pharmaceutically acceptable salts and hydrates unless otherwise understood from the context of the description or expressly limited to one particular form of the compound, i.e., the compound itself, or a pharmaceutically acceptable salt or hydrate thereof.

### DETAILED DESCRIPTION

### Method

A method of producing a visual marking on the exterior of a human or animal body comprising:
implanting a composition at a site in the human or animal body 1 cm or less from the exterior of the human or animal body wherein the composition comprises a polymerisable precursor and a photo-initiator wherein the photo-initiator causes the polymerisable precursor to polymerise where the photo-initiator is in its excited state;
illuminating the site with electromagnetic radiation having a first wavelength of 400 to 1600 nm, wherein absorption of two or more photons of the electromagnetic radiation excites the photo-initiator causing initiation of polymerisation of the polymerisable precursor to form a polymer within the human or animal body, wherein the polymer is visible on the exterior of the human or animal body;
wherein the resultant polymer comprises, consists or consists essentially of one or more of the group consisting of poly(fluorene)s, polyphenylenes, polypyrenes, polyazulenes, polynaphthalenes, Poly(acetylene)s, Poly(p-phenylene vinylene), poly(pyrrole)s, polycarbazoles, polyindoles, polyazepines, poly(thiophene)s, poly(3,4-ethylenedioxythiophene), poly(p-phenylene sulfide), polyanilines and polyfurans; and
wherein the resultant polymer has an electrical conductivity of 10⁻¹⁰ S/cm or more.

According to one embodiment the resultant polymer has an electrical conductivity of 10⁻¹⁰ S/cm to 10⁴ S/cm.

Suitably, polymerisation causes a change in physical state of the polymerizable precursor. Generally, the change in physical state involves solidification of the polymerisable precursor. Alternatively, the viscosity of the polymerisable precursor may be greatly increased, for example to yield a gel (including a hydrogel), paste, foam or plastic. Advantageously, the polymerisation causes solidification of the polymerisable precursor.

Generally, the dimensions of the polymerised area (in general the solidified area) are at least 1 nm, typically at least 100 nm. Typically, the polymerised area has an associated thickness of less than 1 cm.

Currently the provision of structures *in vivo* requires incision and implantation. The present invention is advantageous because it permits *in situ in vivo* fabrication of structures and may require as few as one injection of the precursor materials.

The use of multi-photon technology is advantageous because it may be controlled so that a desired photochemical reaction occurs only at the point of intersection of two or more photon beams. For example two-photon polymerisation may provide point by point scanning to build a desired structure.

The composition is generally implanted through injection. The composition may be injected into the lower layers of the skin, or subcutaneously.

Generally the composition is implanted within 5 mm of the surface of the human or animal body.

According to one embodiment, the composition is implanted to the lower layers of skin, or directly under the skin of the human or animal body.

The closer the site of the implanted composition to the energy source upon illumination of the site by the energy source, the more targeted and controllable the polymerisation of the composition.

Generally, the site of the implanted composition should be no more than 30 cm from the energy source upon illumination of the site, suitably 10 cm or less from the energy source. Typically the site is 5 cm or less from the energy source upon illumination, suitably 1 cm or less from the energy source upon illumination.

To excite the photo-initiator, a threshold amount of radiation must be absorbed. The wavelength of the incident radiation is 400 to 1600 nm.

The threshold incident radiation is generally in the form of at least two photons from intersecting photon beams.

The composition is subjected to electromagnetic radiation from outside the human or animal body to initiate polymerisation of the monomer.

Absorption by the photo-initiator of a threshold amount of electromagnetic radiation excites the photo-initiator, and the excited photo-initiator initiates polymerisation of the polymerisable precursor. Generally, absorption of electromagnetic radiation from the intersection of two or more photon beams is required to excite the photo-initiator, meaning that the photochemical reaction is very controllable and predictable.

The use of multi-photon technology is advantageous because it may be controlled so that a desired photochemical reaction occurs only at the point of intersection of two or more photon beams. For example, two-photon polymerisation may provide point by point scanning to build a desired structure.

Upon polymerisation, the polymerisable precursor undergoes a change in physical state. Generally the polymerisable precursor solidifies. Alternatively, a hydrogel or plastics material may be formed. Generally, the area of polymerisation, of the precursor is within 1,000,000 nm of the incident threshold electromagnetic radiation, typically within 10,000 nm of the intersection of two or more photon beams.

The electromagnetic radiation is generally emitted by an energy source such as a laser.

The dimensions of the polymer formed according to the method of the present invention (width, length, thickness) are generally at least 10 nm, typically 10 nm to 10 cm, suitably 50 nm to 5 cm thick, more suitably 50 nm to 1 cm, advantageously 50 nm to 100 nm.

Generally, the polymer formed is a relatively thin polymer film or coating, and has a relatively large associated length and width. The polymer generally has a thickness of 10 nm to 200 nm, (typically a maximum thickness of 100 nm) and a width and/or length of 1 cm to 5 cm.

According to one embodiment, the polymer formed according to the method of the present invention (width, length, thickness) may have dimensions of from 1 nm to 2 mm.

It is envisaged that the polymer may have dimensions of from 10 cm to 2 m.

Where the polymer has a thickness of more than 1 cm, areas of the composition furthest from an external surface of the human or animal body are generally polymerised before areas of the composition closer to an external surface of the human or animal body by controlling the intersection of the photon beams within the human or animal body.

The polymer formed according to the method of the present invention may be in the form of a polymeric structure.

The polymer formed has an electrical conductivity of at least 10⁻¹⁰ S/cm, typically 10⁻¹⁰ S/cm to 10⁴ S/cm, generally 1 S/cm to 5,000 S/cm, suitably 10 S/cm to 1,000 S/cm.

According to one embodiment, the polymer formed may be a stimulus responsive material. The physical dimensions of the polymer may alter in response to a stimulus, in particular the shape/size/properties (for instance mechanics, optical, electrical properties) may alter in response to the stimulus.

The resultant polymer generally has an electrical conductivity of 10⁻¹⁰ S/cm to 10⁴ S/cm or more, suitably 0.0001 S/cm to 1,000 S/cm; typically, 0.001 S/cm to 1 S/cm.

The resultant polymer is generally one colour, although functional groups may be added to alter the colour of the polymer at different areas.

According to one embodiment, the resultant polymer shows more than one colour on the exterior of the human or animal body.

Generally, the visual marking is in the form of a tattoo.

The composition is suitably injected into the lower surfaces of the skin of the human or animal body. The visual marking may grow out as the skin is shed. Alternatively, the composition may be injected just under the skin of the human or animal body, to provide a permanent visual marking.

The visual marking may be altered or removed using the polymer dis-assembling methods of the present teachings. This can provide significant advantages over current tattooing methods.

The visual marking of the present invention may provide an alternative to branding animals. The visual marking of the present teachings may be altered or removed using the polymer dis-assembling methods of the present teachings, and this can prove useful if the ownership of the animal changes.

### Method of Dis-assembling Polymer

By use of suitable chemistry in the present invention, a first multi-photon reaction (using first functional moieties) may be used to assemble a structure; and a second multi-photon reaction at a different energy (using second functional moieties) may be used later to disassemble the same structure.

According to one embodiment, the composition includes second photo-initiatable functional moieties which become excited at a different wavelength to the photo-initiator. Generally the difference in wavelength is at least 200 nm. The polymerisable precursor generally includes the second photo-initiatable functional moieties, and following polymerisation thereof, the polymer generally comprises the second photo-initiatable functional moieties.

The polymer formed according to the method of the present teachings may be dis-assembled through illumination of the site with electromagnetic radiation having a second wavelength. The second wavelength may be at least 200 nm higher or lower than the first wavelength wherein absorption of two or more photons of the electromagnetic radiation causes the second photo-initiatable functional moieties on the polymer to become excited and destroy the polymer.

The method described herein may include dis-assembling the polymer formed by illuminating the site with electromagnetic radiation having a second wavelength. The second wavelength may be different to the first wavelength by at least 200 nm, wherein absorption of two or more photons of the electromagnetic radiation excites second photo-initiatable functional moieties on the polymer, and the excited second photo-initiatable functional moieties destroy the polymer.

As for the method of forming the polymer, the photo-initiatable destruction is precisely targeted and controllable. Generally, the destruction of the polymer is limited to 5 mm or less, typically 1 mm or less from the location of the incident electromagnetic radiation; typically, from the location of the intersection of two or more photon beams.

Upon destruction of the polymer through the reaction initiated through the excitation of the second photo-initiatable functional moieties, waste products are formed and these are generally non-toxic to the human or animal body. Generally, the waste products are bio-absorbable and may be absorbed by the human or animal body.

Suitable photo-initiatable functional moieties to destroy the polymer include: azobenzene (excitable at around 366 to 420 nm), o-nitrobenzyl (excitable at 350, 750 nm), 3,4-dihydroxycinnamic acid-**co**-4-hydroxycinnamic acid (P(3,4DHCA-co-4HCA)) (at 254, 280 nm), dipalmitoylphosphatidylcholine (at 514 nm), diazonaphthoquinone-based molecules (using UV/NIR).

Generally, the second photo-initiatable functional moieties are initiated at lower wavelengths compared to the wavelength for exciting the photo-initiator.

### Composition

According to an aspect of the present invention, there is provided an injectable composition comprising a polymerisable precursor and a photo-initiator wherein the photo-initiator causes the polymerisable precursor to polymerise where the photo-initiator is in its excited state,
wherein absorption of two or more photons of electromagnetic radiation having a wavelength of 400 nm to 1600 nm excites the photo-initiator causing initiation of polymerisation of the polymerisable precursor,
wherein the resultant polymer comprises, consists or consists essentially of one or more of the group consisting of poly(fluorene)s, polyphenylenes, polypyrenes, polyazulenes, polynaphthalenes, poly(acetylene)s, poly(p-phenylene vinylene), poly(pyrrole)s, polycarbazoles, polyindoles, polyazepines, poly(thiophene)s, poly(3,4-ethylenedioxythiophene), poly(p-phenylene sulfide), polyanilines and polyfurans,
wherein the resultant polymer has an associated electrical conductivity of at least 10⁻¹⁰ S/cm or more.

According to one embodiment the resultant polymer has an electrical conductivity of 10⁻¹⁰ S/cm to 10⁴ S/cm.

The composition generally includes a visual indicator which alters upon polymerisation of the polymerisable precursor. Suitable visual indicators include dyes. Generally, the visual indicator is visible in the polymerisable precursor but not in the polymer. Alternatively, the visual indicator may be visible in the polymer but not the polymerisable precursor.

The visual indicator may be visible upon illumination with UV light.

The visual indicator may change colour between monomer and polymer.

The incorporation of a visual indicator which alters upon polymerisation of the polymerisable precursor is useful following implantation of the composition into a human or animal body. Specifically, it can assist in showing the precise location of the composition.

Generally said composition has a viscosity in the range from 1 centipoise (similar to water), to 250,000 centipoise (similar to peanut butter). A viscosity of 1 centipoise or more helps to ensure the injected composition is retained in a predictable position (generally at the site of injection). A viscosity of 250,000 centipoise or less reduces the risk of blockages within the human or animal body, which can be of paramount importance where the composition is implanted near a blood vessel.

Typically, the composition is buffered to a pH of 4-10, but most commonly pH 7.4. Generally, the composition is sterilised prior to implantation.

The polymerisable precursor may comprise, consist or consist essentially of monomers having at least one olefinic bond, oligomers having at least one olefinic bond, polymers having at least one olefinic bond, olefins, halogenated olefins, acrylates, methacrylates, pyrroles, acrylamides, bisacrylamides, styrenes, epoxides, cyclohexeneoxide, amino acids, peptides, proteins, fatty acids, lipids, nucleotides, oligonucleotides, synthetic nucleotide analogues, nucleic acids, sugars, carbohydrates, cytokines, hormones, receptors, growth factors, drugs, and mixtures thereof.

The composition generally comprises 5-50 wt.% polymerisable precursor.

Generally, the polymer is an intrinsically conductive polymer. The polymer generally includes repeating units comprising at least one aromatic cycle, typically a 5 to 7 member aromatic cycle, optionally including one or more heteroatoms, in particular one or more N or S heteroatoms. The repeating units may include one or more N, S or O atoms outside the aromatic cycle.

The polymer generally includes repeating units comprising at least one double bond.

Typically the polymer includes repeating units comprising more than one aromatic cycle, repeating units comprising an aromatic cycle and a double bond or repeating units comprising an aromatic cycle and one or more N, S or O atoms outside the aromatic cycle.

Alternatively, the polymer may be a block copolymer with conductive blocks, hyperbranched polymers, dendrimers, supramolecular polymers, where the conductive blocks are derivatives of derivatives of pyrrole, aniline, thiophene, 3,4-ethylenedioxythiophene, fluorene, phenylene, pyrene, azulene, napthalene, indole, azepine, p-phenylene sulfide, p-phenylene vinylene, and/or furans.

The polymer is biocompatible.

Generally the polymer itself has a relatively high associated conductivity (10⁻⁴ S/cm to 10⁴ S/cm).

Alternatively, the composition may include a polymerisable precursor suitable to form a polymer having a relatively low electrical conductivity such as ceramics or glasses and an electrically conductive material dispersed throughout. Suitable such conductive material includes inorganic metals and alloys such as Au, Ti, Steel, CoCr, CoCrMo, Ti₆Al₄V, Ti₆Al₇Nb, iron, WE43 magnesium alloy, Mg/Zn.

The composition of the present teachings includes a polymerisable precursor and a photo-initiator.

The polymerisable precursor itself may comprise the photo-initiator, in particular, the polymeric precursor may be functionalised with photoactivatable groups.

Alternatively, or additionally, the photo-initiator may be provided separately from the polymerisable precursor.

According to one embodiment, the polymerisable precursor may be encapsulated within, or coated with the photo-initiator.

Any suitable photo-initiator may be used. Mention may be made of azo compounds, azobisisobutyronitrile, peroxides, benzoyl peroxide, aliphatic ketones and diketones, aromatic diketones, benzophenone, 9-fluorenone 2-carboxylic acid, ion pairs, the ion pair Fe3⁺OH⁻, the ion pair Pb2⁺Cl⁻), photosensitive dyes, eosin, rose Bengal, erydirosin, photosensitive transition metal derivatives, and Mn₂(CO)₁₀ in the presence of organic halides, triarylsulfonium salts with complex metal halide anions, diaryliodonium salts with complex metal halide anions, mixed arene cyclopentadienyl metal salts of complex metal halide anions, and (6-benzene)(5-cyclopentadienyl)Fe(II) hexafluorophosphate.

The composition generally comprises 1-10 wt.% photo-initiator.

The composition generally includes second photo-initiatable functional moieties which become excited at a different wavelength to the photo-initiator. Generally the difference in wavelength is at least 200 nm.

Other photo-initiation systems include, but are not limited to, redox-type photo-initiators useful in aqueous systems (e.g., ion pairs such as Fe³⁺OH⁻, and Pb²⁺Cl⁻), photosensitive dyes such as eosin, rose Bengal, and erythrosin, and transition metal derivatives such as Mn₂(CO)₁₀ in the presence of organic halides, riboflavin/triethanolamine, Irgacure 184, Irgacure 369, Irgacure 651, Irgacure 2959, azobisisobutyronitrile, peroxides such as benzoyl peroxide, aliphatic carbonyl compounds such as ketones and diketones, and aromatic diketones such as benzophenone and its derivatives, and 9-fluorenone 2-carboxylic acid.

The polymerisable precursor may comprise the second photo-initiatable functional moieties.

Generally, the composition is aqueous, although in some embodiments non-aqueous compositions may be of utility.

The composition is generally in the form of a gel, cream, liquid, suspension, solution, emulsion or foam. Typically, the composition is in the form of an injectable gel. Suitable gel based carriers include synthetic polymers (e.g. polyethylene glycol) and biopolymers (e.g. polysaccharides [hyaluronic acid, chitin, chitosan, alginate, cellulose], proteins [collagen, fibronectin, silk, etc.], lignins, polynucleotides, extracellular matrix).

The composition may suitably comprise 5-25% gel based carrier.

According to one embodiment, the composition includes particles in an injectable carrier material, said particles comprising a core having an electrical conductivity of at least 10⁻¹⁰ S/cm to 10⁴ S/cm, and a coating comprising the photo-initiator.

The composition generally comprises polymerisable precursors suitable for forming a polymer having an electrical conductivity of at least 10⁻¹⁰ S/cm, a photo-initiator and a dye.

According to a further aspect of the present invention, there is provided a buffered aqueous based composition comprising derivatives of pyrrole, aniline, thiophene, 3,4-ethylenedioxythiophene, fluorene, phenylene, pyrene, azulene, napthalene, indole, azepine, p-phenylene sulfide, p-phenylene vinylene, and/or furans, a photo-initiator, a dye.

According to a further aspect of the present invention there is provided a buffered aqueous based composition comprising pyrrole, a photo-initiator (generally irgacure) and a dye.

The present invention will now be described by way of example only, with reference to the accompanying Figures, in which:
Figure 1 provides a schematic representation of the manufacture of a subcutaneous structure according to the present invention. (not to scale);
Figure 2 provides a schematic representation of assembly and disassembly of polymers according to the present invention;
Figure 3 is a halftone rendering of a photograph of a polymeric structure printed into vertebrate tissue.

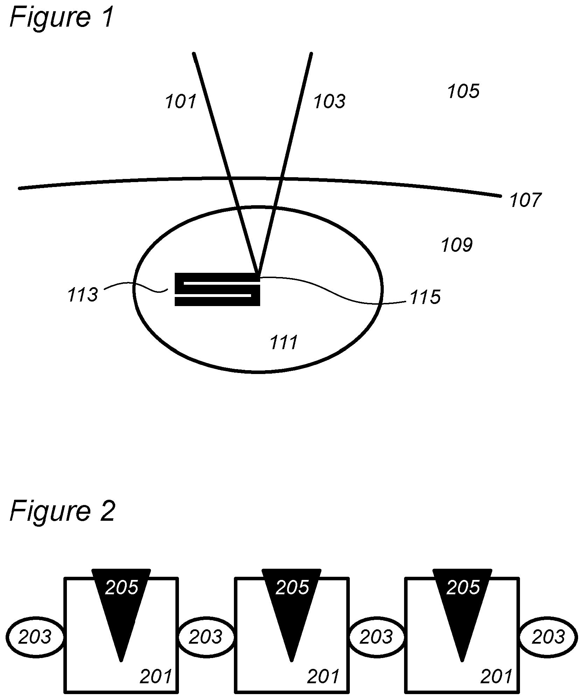

Figure 1 shows schematically and not to scale the manufacture of a subcutaneous device according to the present invention. A first photon beam (101) and a second photon beam (103) pass from outside (105) tissue, through the surface (107) of tissue, into the interior of tissue (109). Previously a region of precursor material (111) has been placed into the tissue (109). The photon beams cause polymerisation of precursor monomers at the point of intersection (115), and when the beams are moved, this point (115) moves and a polymerised structure (113) results.

Figure 2 shows schematically assembly and disassembly of polymers according to the present invention. Each square block (201) represents a monomer unit that has polymerized via a multi-photon polymerisation reaction creating a bond (203) to a neighbouring monomer unit (201). In this embodiment each monomer also comprises a suitably designed moiety (205; represented by a triangle), via which a second multi-photon process may disassemble the polymer when required.

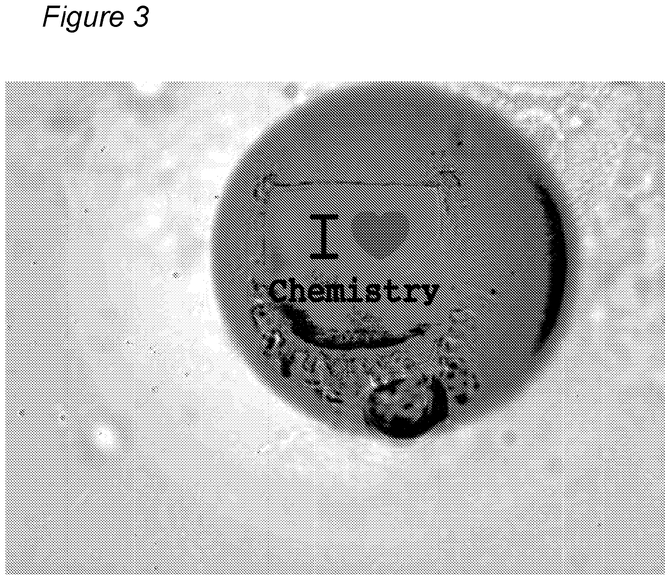

A composition of the present invention, was provided including pyrrole as the polymerisable precursor and irgacure as the photo-initiator. The composition was injected under the skin of a chicken breast. The external surface of the chicken breast was illuminated with two laser beams, and at the location of intersection, the photo-initiator was excited causing localised polymerisation of the pyrrole to form polypyrrole. The lasers were used to controllably initiate polymerisation in a targeted manner. The polypyrrole is shown in Figure 3 as the dark lines which read "I heart Chemistry".

## Claims

1. A method of producing a visual marking on the exterior of a human or animal body comprising:
implanting a composition at a site in the human or animal body 1 cm or less from the exterior of the human or animal body wherein the composition comprises a polymerisable precursor and a photo-initiator wherein the photo-initiator causes the polymerisable precursor to polymerise where the photo-initiator is in its excited state,
illuminating the site with electromagnetic radiation having a wavelength of 400 to 1600 nm, wherein absorption of two or more photons of the electromagnetic radiation excites the photo-initiator causing initiation of polymerisation of the polymerisable precursor to form a polymer within the human or animal body, wherein the polymer is visible on the exterior of the human or animal body;
wherein the resultant polymer comprises, consists or consists essentially of one or more of the group consisting of poly(fluorene)s, polyphenylenes, polypyrenes, polyazulenes, polynaphthalenes, Poly(acetylene)s, Poly(p-phenylene vinylene), poly (pyrrol e)s, polycarbazoles, polyindoles, polyazepines, poly(thiophene)s, poly(3,4-ethylenedioxythiophene), poly(p-phenylene sulfide), polyanilines and polyfurans; and
wherein the resultant polymer has an electrical conductivity of 10⁻¹⁰ S/cm or more.

2. A method as claimed in Claim 1 wherein the resultant polymer shows more than one colour on the exterior of the human or animal body.

3. A method as claimed in either one of Claims 1 and 2 wherein the visual marking is in the form of a tattoo.

4. A method as claimed in either one of Claims 1 and 2 wherein the composition is injected into the lower surfaces of the skin of the human or animal body and the visual marking grows out as the skin is shed.

5. A method as claimed in either one of Claims 1 and 2 wherein the composition is injected under the skin of the human or animal body, to provide a permanent visual marking.

6. A method as claimed in any preceding Claim wherein the threshold incident radiation is in the form of at least two photons from intersecting photon beams.

7. A method as claimed in any preceding Claim wherein the composition includes second photo-initiatable functional moieties which become excited at a second wavelength different to the first wavelength wherein the polymer is dis-assembled through illumination of the site with electromagnetic radiation having the second wavelength, wherein absorption of two or more photons of the electromagnetic radiation causes the second photo-initiatable functional moieties on the polymer to become excited and disassemble the polymer.

8. A method as claimed in Claim 7 wherein the second wavelength is at least 200 nm higher or lower than the first wavelength.

9. An injectable composition comprising a polymerisable precursor and a photo-initiator wherein the photo-initiator causes the polymerisable precursor to polymerise where the photo-initiator is in its excited state,
wherein absorption of two or more photons of electromagnetic radiation having a wavelength of 400 nm to 1600 nm excites the photo-initiator causing initiation of polymerisation of the polymerisable precursor,
wherein the resultant polymer comprises, consists or consists essentially of one or more of the group consisting of poly(fluorene)s, polyphenylenes, polypyrenes, polyazulenes, polynaphthalenes, poly(acetylene)s, poly(p-phenylene vinylene), poly (pyrrol e)s, polycarbazoles, polyindoles, polyazepines, poly(thiophene)s, poly(3,4-ethylenedioxythiophene), poly(p-phenylene sulfide), polyanilines and polyfurans; and
wherein the resultant polymer has an associated electrical conductivity of at least 10⁻¹⁰ S/cm.

10. A composition as claimed in Claim 9 including a visual indicator which alters upon polymerisation of the polymerisable precursor, wherein the visual indicator is visible in the polymerisable precursor but not in the polymer, or the visual indicator is visible in the polymer but not the polymerisable precursor.

11. A composition as claimed in either one of Claims 9 and 10 having a viscosity of from 1 centipoise to 250,000 centipoise.

12. A composition as claimed in any one of Claims 9 to 11, wherein the polymerisable precursor comprising, consisting or consisting essentially of monomers having at least one olefinic bond, oligomers having at least one olefinic bond, polymers having at least one olefinic bond, olefins, halogenated olefins, acrylates, methacrylates, pyrroles, acrylamides, bisacrylamides, styrenes, epoxides, cyclohexeneoxide, amino acids, peptides, proteins, fatty acids, lipids, nucleotides, oligonucleotides, synthetic nucleotide analogues, nucleic acids, sugars, carbohydrates, cytokines, hormones, receptors, growth factors, drugs, and mixtures thereof.

## Patentansprüche

1. Verfahren zum Erzeugen einer visuellen Markierung an der Außenseite eines menschlichen oder tierischen Körpers, umfassend:
Implantieren einer Zusammensetzung an einer Stelle in dem menschlichen oder tierischen Körper 1 cm oder weniger von der Außenseite des menschlichen oder tierischen Körpers entfernt, wobei die Zusammensetzung einen polymerisierbaren Vorläufer und einen Photoinitiator umfasst, wobei der Photoinitiator bewirkt, dass der polymerisierbare Vorläufer dort polymerisiert, wo sich der Photoinitiator in seinem angeregten Zustand befindet,
Beleuchten der Stelle mit elektromagnetischer Strahlung mit einer Wellenlänge von 400 bis 1600 nm, wobei die Absorption von zwei oder mehr Photonen der elektromagnetischen Strahlung den Photoinitiator anregt, wodurch die Polymerisation des polymerisierbaren Vorläufers initiiert wird, um ein Polymer in dem menschlichen oder tierischen Körper auszubilden; wobei das Polymer an der Außenseite des menschlichen oder tierischen Körpers sichtbar ist;
wobei das resultierende Polymer eine oder mehrere der Gruppen umfasst, aus diesen besteht oder im Wesentlichen aus diesen besteht, die aus Poly(fluoren)en, Polyphenylenen, Polypyrenen, Polyazulenen, Polynaphthalinen, Poly(acetylen)en, Poly(p-phenylenvinylen), Poly(pyrrol)en, Polycarbazolen, Polyindolen, Polyazepinen, Poly(thiophen)en, Poly(3,4-ethylendioxythiophen), Poly(p-phenylensulfid), Polyanilinen und Polyfuranen besteht; und
wobei das resultierende Polymer eine elektrische Leitfähigkeit von 10⁻¹⁰ S/cm oder mehr aufweist.

2. Verfahren nach Anspruch 1, wobei das resultierende Polymer mehr als eine Farbe an der Außenseite des menschlichen oder tierischen Körpers zeigt.

3. Verfahren nach einem der Ansprüche 1 und 2, wobei die visuelle Markierung in der Form einer Tätowierung vorliegt.

4. Verfahren nach einem der Ansprüche 1 und 2, wobei die Zusammensetzung in die unteren Oberflächen der Haut des menschlichen oder tierischen Körpers injiziert wird und die visuelle Markierung herauswächst, wenn die Haut abgestoßen wird.

5. Verfahren nach einem der Ansprüche 1 und 2, wobei die Zusammensetzung unter die Haut des menschlichen oder tierischen Körpers injiziert wird, um eine dauerhafte visuelle Markierung bereitzustellen.

6. Verfahren nach einem der vorhergehenden Ansprüche, wobei die einfallende Schwellenstrahlung in der Form von wenigstens zwei Photonen von sich kreuzenden Photonenstrahlen vorliegt.

7. Verfahren nach einem der vorhergehenden Ansprüche, wobei die Zusammensetzung zweite photoinitiierbare funktionelle Reste umfasst, die bei einer zweiten Wellenlänge angeregt werden, die sich von der ersten Wellenlänge unterscheidet, wobei das Polymer durch Beleuchten der Stelle mit elektromagnetischer Strahlung mit der zweiten Wellenlänge zerlegt wird, wobei die Absorption von zwei oder mehr Photonen der elektromagnetischen Strahlung bewirkt, dass die zweiten photoinitiierbaren funktionellen Reste auf dem Polymer angeregt werden und das Polymer zerlegen.

8. Verfahren nach Anspruch 7, wobei die zweite Wellenlänge wenigstens 200 nm höher oder niedriger als die erste Wellenlänge ist.

9. Injizierbare Zusammensetzung, umfassend einen polymerisierbaren Vorläufer und einen Photoinitiator, wobei der Photoinitiator bewirkt, dass der polymerisierbare Vorläufer dort polymerisiert, wo sich der Photoinitiator in seinem angeregten Zustand befindet;
wobei die Absorption von zwei oder mehr Photonen elektromagnetischer Strahlung mit einer Wellenlänge von 400 nm bis 1600 nm den Photoinitiator anregt, wodurch die Polymerisation des polymerisierbaren Vorläufers initiiert wird;
wobei das resultierende Polymer eine oder mehrere der Gruppen umfasst, aus diesen besteht oder im Wesentlichen aus diesen besteht, die aus Poly(fluoren)en, Polyphenylenen, Polypyrenen, Polyazulenen, Polynaphthalinen, Poly(acetylen)en, Poly(p-phenylenvinylen), Poly(pyrrol)en, Polycarbazolen, Polyindolen, Polyazepinen, Poly(thiophen)en, Poly(3,4-ethylendioxythiophen), Poly(p-phenylensulfid), Polyanilinen und Polyfuranen besteht; und wobei das resultierende Polymer eine zugehörige elektrische Leitfähigkeit von wenigstens 10⁻¹⁰ S/cm aufweist.

10. Zusammensetzung nach Anspruch 9, einschließlich eines visuellen Indikators, der sich bei der Polymerisation des polymerisierbaren Vorläufers ändert, wobei der visuelle Indikator in dem polymerisierbaren Vorläufer sichtbar ist, jedoch nicht in dem Polymer, oder der visuelle Indikator in dem Polymer sichtbar ist, jedoch nicht in dem polymerisierbaren Vorläufer.

11. Zusammensetzung nach einem der Ansprüche 9 und 10 mit einer Viskosität von 1 Centipoise bis 250.000 Centipoise.

12. Zusammensetzung nach einem der Ansprüche 9 bis 11, wobei der polymerisierbare Vorläufer, der Monomere mit wenigstens einer olefinischen Bindung, Oligomere mit wenigstens einer olefinischen Bindung, Polymere mit wenigstens einer olefinischen Bindung, Olefine, halogenierte Olefine, Acrylate, Methacrylate, Pyrrole, Acrylamide, Bisacrylamide, Styrole, Epoxide, Cyclohexenoxid, Aminosäuren, Peptide, Proteine, Fettsäuren, Lipide, Nukleotide, Oligonukleotide, synthetische Nukleotidanaloga, Nukleinsäuren, Zucker, Kohlenhydrate, Cytokine, Hormone, Rezeptoren, Wachstumsfaktoren und Mischungen davon umfasst, aus diesen besteht oder im Wesentlichen aus diesen besteht.

## Revendications

1. Procédé de production d'un marquage visuel sur l'extérieur d'un corps humain ou animal comprenant :
l'implantation d'une composition au niveau d'un site dans le corps humain ou animal à 1 cm ou moins de l'extérieur du corps humain ou animal dans lequel la composition comprend un précurseur polymérisable et un photoamorceur dans lequel le photoamorceur amène le précurseur polymérisable à se polymériser là où le photoamorceur est dans son état excité,
l'illumination du site avec un rayonnement électromagnétique ayant une longueur d'onde allant de 400 à 1 600 nm, dans lequel l'absorption de deux photons ou plus du rayonnement électromagnétique excite le photoamorceur provoquant l'amorçage de la polymérisation du précurseur polymérisable pour former un polymère au sein du corps humain ou animal, dans lequel le polymère est visible à l'extérieur du corps humain ou animal ;
dans lequel le polymère qui en résulte comprend, est constitué ou est constitué essentiellement par un ou plusieurs éléments parmi le groupe constitué par des poly(fluorène)s, des polyphénylènes, des polypyrènes, des polyazulènes, des polynaphtalènes, des poly(acétylène)s, des poly(p-phénylène vinylène), des poly(pyrrole)s, des polycarbazoles, des polyindoles, des polyazépines, des poly(thiophène)s, des poly(3,4-éthylènedioxythiophène), des poly(sulfure de p-phénylène), des polyanilines et des polyfuranes ; et
dans lequel le polymère qui en résulte a une conductivité électrique de 10⁻¹⁰ S/cm ou plus.

2. Procédé selon la revendication 1 dans lequel le polymère qui en résulte révèle plus d'une couleur sur l'extérieur du corps humain ou animal.

3. Procédé selon l'une ou l'autre des revendications 1 et 2 dans lequel le marquage visuel est sous la forme d'un tatouage.

4. Procédé selon l'une ou l'autre des revendications 1 et 2 dans lequel la composition est injectée dans les surfaces inférieures de la peau du corps humain ou animal et le marquage visuel disparaît au fur et à mesure que la peau est éliminée.

5. Procédé selon l'une ou l'autre des revendications 1 et 2 dans lequel la composition est injectée sous la peau du corps humain ou animal, pour fournir un marquage visuel permanent.

6. Procédé selon l'une quelconque des revendications précédentes dans lequel le rayonnement incident seuil est sous la forme d'au moins deux photons de faisceaux de photons qui s'entrecoupent.

7. Procédé selon l'une quelconque des revendications précédentes dans lequel la composition inclut des deuxièmes groupements fonctionnels capables de photoamorçage qui deviennent excités à une deuxième longueur d'onde différente de la première longueur d'onde dans lequel the polymère est désassemblé par l'illumination du site avec un rayonnement électromagnétique ayant la deuxième longueur d'onde, dans lequel l'absorption de deux photons ou plus du rayonnement électromagnétique amène les deuxièmes groupements fonctionnels photoamorçables sur le polymère à devenir excités et à désassembler le polymère.

8. Procédé selon la revendication 7 dans lequel la deuxième longueur d'onde est supérieure ou inférieure d'au moins 200 nm à la première longueur d'onde.

9. Composition injectable comprenant un précurseur polymérisable et un photoamorceur dans laquelle le photoamorceur amène le précurseur polymérisable à se polymériser là où le photoamorceur est dans son état excité,
dans laquelle l'absorption de deux photons ou plus de rayonnement électromagnétique ayant une longueur d'onde de 400 nm à 1 600 nm excite le photoamorceur provoquant l'amorçage de la polymérisation du précurseur polymérisable,
dans laquelle le polymère qui en résulte comprend, est constitué ou est constitué essentiellement par un ou plusieurs éléments parmi le groupe constitué par des poly(fluorène)s, des polyphénylènes, des polypyrènes, des polyazulènes, des polynaphtalènes, des poly(acétylène)s, des poly(p-phénylène vinylène), des poly(pyrrole)s, des polycarbazoles, des polyindoles, des polyazépines, des poly(thiophène)s, des poly(3,4-éthylènedioxythiophène), des poly(sulfure de p-phénylène), des polyanilines et des polyfuranes ; et
dans laquelle le polymère qui en résulte a une conductivité électrique associée d'au moins 10⁻¹⁰ S/cm.

10. Composition selon la revendication 9 incluant un indicateur visuel qui change à la polymérisation du précurseur polymérisable, dans laquelle l'indicateur visuel est visible dans le précurseur polymérisable mais pas dans le polymère, ou l'indicateur visuel est visible dans le polymère mais pas le précurseur polymérisable.

11. Composition selon l'une ou l'autre des revendications 9 et 10 présentant une viscosité de 1 centipoise à 250 000 centipoises.

12. Composition selon l'une quelconque des revendications 9 à 11, dans laquelle le précurseur polymérisable comprend, est constitué ou est constitué essentiellement par des monomères ayant au moins une liaison oléfinique, des oligomères ayant au moins une liaison oléfinique, des polymères ayant au moins une liaison oléfinique, des oléfines, des oléfines halogénées, des acrylates, des méthacrylates, des pyrroles, des acrylamides, des bisacrylamides, des styrènes, des époxydes, un oxyde de cyclohexène, des acides aminés, des peptides, des protéines, des acides gras, des lipides, des nucléotides, des oligonucléotides, des analogues de nucléotides synthétiques, des acides nucléiques, des sucres, des hydrates de carbone, des cytokines, des hormones, des récepteurs, des facteurs de croissance, des médicaments, et des mélanges de ceux-ci.
